# EUROPEAN PATENT APPLICATION

(11) **EP 4 818 699 A1**
(43) Date of publication of application: **07.10.2026**
(21) Application number: 26170329.2
(22) Date of filing: 01.04.2026
(51) Int. Cl.: G01N 33/52, G01N 33/68, G01N 33/72, G01N 21/77

(54) **VOLUME CONTROL TEST STRIP**

(30) Priority: 01.04.2025 US 202563781555 P
(71) Applicant: Jana Care, Inc., Watertown, MA 02472 (US)
(72) Inventor: Grover, Kanishka, Watertown 02472 (US); Williams, Richard E., Watertown 02472 (US); Prokopis, Mitchell R., Watertown 02472 (US)
(74) Representative: Marks & Clerk LLP

(57) **Abstract**

A test strip includes a top layer, a defined blood volume provision layer, and a sample pad. The defined blood volume provision layer is beneath the top layer. The defined blood volume provision layer includes a defined blood volume provision channel. A terminal end of the defined blood volume provision channel has a terminal opening at a bottom surface. The sample pad is beneath the terminal opening and adapted to receive fluid from the terminal opening. Contacting a finger-prick blood droplet with a sealed engagement opening causes the channel to fill with a precise volume of the blood. The blood remains in the channel while contact between the finger-prick blood droplet and the channel is maintained. The blood flows into the sample pad following separation between the finger-prick blood droplet and the channel. The channel is adapted to accommodate a range of contact pressures and a range of contact durations.

## Description

### CROSS-REFERENCE TO RELATED APPLICATION

This application claims priority to and the benefit of U.S. Provisional Application No. 63/781,555 filed in the U.S. Patent and Trademark Office on April 1, 2025. The foregoing application is incorporated by reference for all purposes.

### BACKGROUND

Historically, at-home blood testing that requires a known and small volume of blood has been unattainable due to the lack of an adequate device for securing the known and small volume under the highly variable conditions that an at-home user is expected to use the device. Instead, the industry still relies on medical professionals securing the known and small volume of blood for testing, thereby maintaining barriers to care in circumstances where the assistance of medical professionals is not feasible.

Recently, some have approached this problem by developing highly complex and sophisticated devices for securing a known and small volume of blood. In one instance, Captainer B (https://capitainer.com/technology/) utilizes a "double valve solution" involving dissolvable membranes to collect a known and small volume of blood (albeit for a dried blood measurement). In another instance, a commercial device from Now Diagnostics (https://nowdx.com) claims to achieve specific volumes of blood plasma (filtering out red blood cells, among other whole blood elements), but utilizes filtration membranes and complex physical structures, including multiple chambers having specific volumes and vents, which can only be presently achieved with more complex and expensive manufacturing methods (e.g., injection molding).

As of the time of filing, when a test requires a specific known and small volume of blood, a medical professional of some degree remains required to administer the test.

With respect specifically to HbA1c testing, in addition to the above-referenced shortcomings, current tests on the market utilize the boronate affinity method with a solvent washing step to eliminate unbound dye. Such a solvent washing step involves additional chemicals and method complexity, which are generally unpreferred for user-administered at-home testing.

A need exists for an at-home testing solution for tests that require a specific known and small volume of blood. A need exists for an at-home testing solution or HbA1c that does not require additional solvents or solvent washing steps.

### SUMMARY

In an aspect, the present disclosure provides a test strip for at home use. The test strip includes a top layer, a defined blood volume provision layer, and a sample pad. The defined blood volume provision layer is beneath the top layer. The defined blood volume provision layer includes a defined blood volume provision channel. The defined blood volume provision channel has a blood droplet engagement end and a terminal end. The defined blood volume provision channel has a sealed engagement opening. The defined blood volume provision channel has at least one hydrophilic inner surface. The terminal end has a terminal opening at a bottom surface of the defined blood volume provision channel. The sample pad is beneath the terminal opening and adapted to receive fluid from the terminal opening. The sample pad optionally comprises chemical agents in dry form. Contacting a finger-prick blood droplet with the sealed engagement opening, such as a user contacting a finger-prick blood droplet with the sealed engagement opening, causes the defined blood volume provision channel to fill with a precise volume of the blood. The blood remains in the defined blood volume provision channel while contact between the finger-prick blood droplet and the sealed engagement opening is maintained. The blood flows into the sample pad following separation between the finger-prick blood droplet and the sealed engagement opening. The defined blood volume provision channel is adapted to accommodate a range of contact pressures and a range of contact durations.

In another aspect, the present disclosure provides a hemoglobin A1c (HbA1c) test strip for at-home use. The HbA1c test strip includes a top layer, a defined blood volume provision layer, a sample pad, and a dye separation material. The defined blood volume provision layer is beneath the top layer. The defined blood volume provision layer includes a defined blood volume provision channel. The defined blood volume provision channel has a blood droplet engagement end and a terminal end. The defined blood volume provision channel has a sealed engagement opening. The defined blood volume provision channel has at least one hydrophilic inner surface. The terminal end has a terminal opening at a bottom surface of the defined blood volume provision channel. The sample pad is beneath the terminal opening and adapted to receive fluid from the terminal opening. The sample pad optionally comprises chemical agents in dry form. Contacting a finger-prick blood droplet with the sealed engagement opening, such as a user contacting a finger-prick blood droplet with the sealed engagement opening, causes the defined blood volume provision channel to fill with a precise volume of the blood. The blood remains in the defined blood volume provision channel while contact between the finger-prick blood droplet and the sealed engagement opening is maintained. The blood flows into the sample pad following separation between the finger-prick blood droplet and the sealed engagement opening. The defined blood volume provision channel is adapted to accommodate a range of contact pressures and a range of contact durations. The dye separation material is in fluid contact with the sample pad, such that fluid from the sample pad flow into the dye separation material. The dye separation material has different migration speeds for HbA1c having the boronic acid dye bound thereto and unbound boronic acid dye.

In yet another aspect, the present disclosure provides a method of making a test strip. The method includes the following steps: a) assembling a layered structure including: a base layer; a sample pad and optional dye separation layer positioned atop the base layer, the sample pad and optional dye separation layer comprising a sample pad and optionally a dye separation material; optional spacer layers and optional spacers within the other layers; a defined blood volume provision layer positioned atop the sample pad and dye separation layer, the defined blood volume provision layer comprising a plurality of defined blood volume provision channels each having a blood droplet engagement end and a terminal end, wherein the blood droplet engagement end has a sealed engagement opening, wherein the terminal end has a terminal opening at a bottom surface of the defined blood volume provision channel; and a top layer positioned atop the defined blood volume provision layer; and b) cutting the layered structure into individual test strips, wherein each individual test strip includes at least one defined blood volume provision channel.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention.
Fig. 1 shows an expanded view of a test strip, in accordance with aspects of the present disclosure.
Fig. 2 shows a schematic of a test strip, in accordance with aspects of the present disclosure.
Fig. 3A shows a cross-section view of a test strip, in accordance with aspects of the present disclosure.
Fig. 3B shows a zoomed in view of a test strip, in accordance with aspects of the present disclosure.
Fig. 4 shows a top-down view of a test strip, in accordance with aspects of the present disclosure.
Fig. 5 shows an expanded view of a test strip, in accordance with aspects of the present disclosure.
Fig. 6 shows a schematic of a test strip, in accordance with aspects of the present disclosure.
Fig. 7A shows a cross-section view of a test strip, in accordance with aspects of the present disclosure.
Fig. 7B shows a zoomed in view of a test strip, in accordance with aspects of the present disclosure
Fig. 8 shows a top-down view of a test strip, in accordance with aspects of the present disclosure.

### DETAILED DESCRIPTION

Before the present disclosure is described in further detail, it is to be understood that the disclosure is not limited to the embodiments described. It is also understood that the terminology used herein is for the purpose of describing particular embodiments only and is not intended to be limiting. As used herein, the singular forms "a", "an", and "the" include plural embodiments unless the context clearly dictates otherwise.

It should be apparent to those skilled in the art that many additional modifications beside those already described are possible without departing from the inventive concepts. In interpreting this disclosure, all terms should be interpreted in the broadest possible manner consistent with the context. Variations of the term "comprising" or "including" should be interpreted as referring to elements, components, or steps in a non-exclusive manner, so the referenced elements, components, or steps may be combined with other elements, components, or steps that are not expressly referenced. When two or more ranges for a particular value are recited, this disclosure contemplates all combinations of the upper and lower bounds of those ranges that are not explicitly recited. For example, recitation of a value of between 1 and 10 or between 2 and 9 also contemplates a value of between 1 and 9 or between 2 and 10. All ranges are inclusive of the upper and lower value.

The present disclosure provides a test strip. The test strip provides a known and defined volume of blood for testing purposes. Compared with current market solutions, the test strip disclosed herein is efficient in design and performance.

Many of the specifics of the device will be described herein in the context of an HbA1C test, but a skilled artisan will recognize that the front-end of the test strip described for this specific context could be deployed in a different technical context, where different reagents are present in the sample pad and different downstream processing of the blood is required. As such, the skilled artisan would recognize the disclosure of a test strip comprising a top layer, a defined blood volume provision layer, a sample pad, and downstream processing components. Any component described within the generic context of the test strip with generic downstream processing components is applicable to any one of the specific testing contexts with a set of specific downstream processing components unless the context clearly dictates otherwise. Similarly, any component described within the context of a specific texting context (i.e., an HbA1c test) is applicable to the generic context of the test strip with generic downstream process unless the context clearly dictates otherwise.

In one aspect, the downstream components are the downstream components of an HbA1c test, as described below. In another aspect, the downstream components are the downstream components of a creatinine test, as would be understood by a person having ordinary skill in the art. In another aspect, the downstream components are the downstream components of a potassium test, as would be understood by a person having ordinary skill in the art. In another aspect, the downstream components are the downstream components of an N-Terminal Pro-B-Type Natriuretic Peptide (NT-proBNP) test, as would be understood by a person having ordinary skill in the art. In another aspect, the downstream components are the downstream components of an aspartate aminotransferase (AST) test, as would be understood by a person having ordinary skill in the art. In another aspect, the downstream components are the downstream components of an alanine aminotransferase (ALT) test, as would be understood by a person having ordinary skill in the art. In another aspect, the downstream components are the downstream components of other blood-specific tests, as would be understood by a person having ordinary skill in the art.

The test strips disclosed herein are contemplated for use in a variety of contexts. In some cases, the test strips may be visual identification test strips, where the result of the test may be read visually, such as where a user can visually read the result of the test. In some cases, the test strips may require a device for measuring one or more properties (e.g., a spectroscopic measurement).

A1c measurements are essential in monitoring blood glucose levels over time. Broadly, glucose binds irreversibly to hemoglobin. A1c tests measure the percentage of hemoglobin with glucose bound to it, referred to as glycated hemoglobin. The present disclosure relates to a hemoglobin A1c (HbA1c) test strip for at-home use.

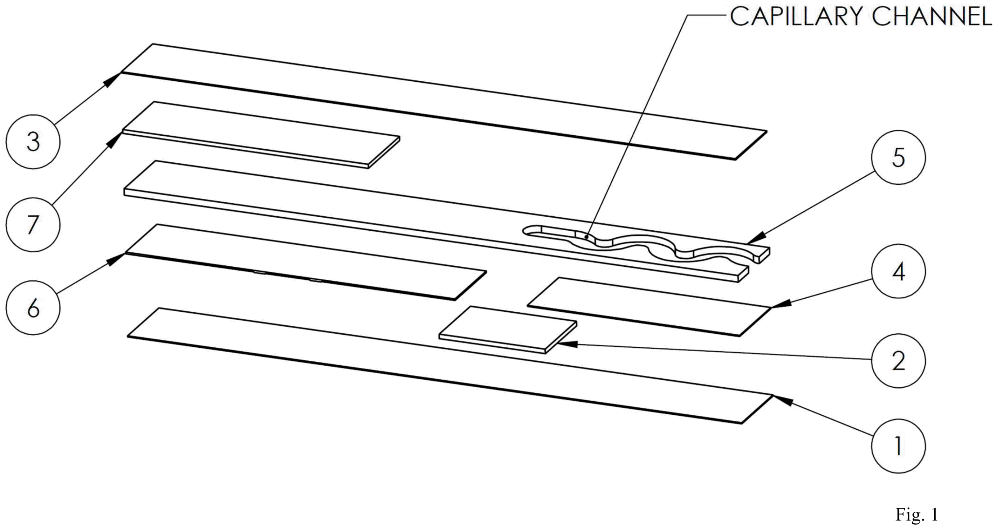

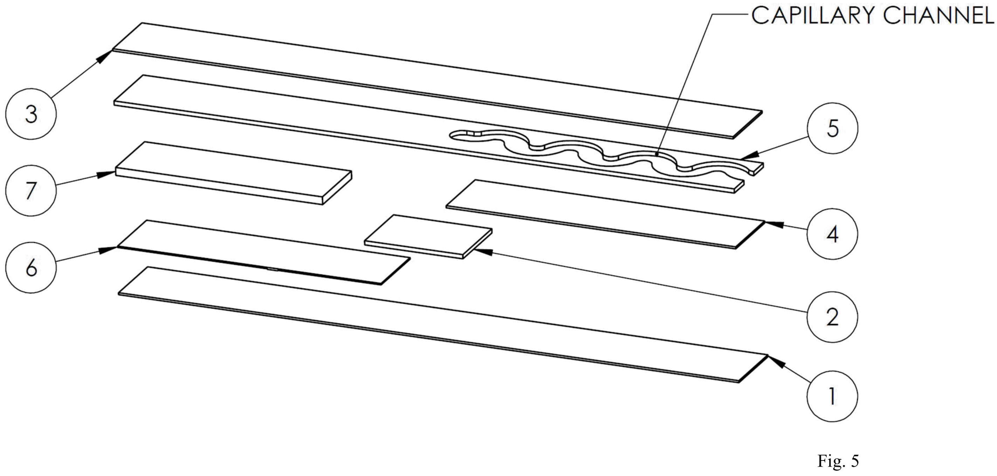

At a high level of one aspect, the test strip may be held to a blood drop. For example, a user can prick their finger and hold the test strip to the blood drop. A channel within the test strip, the defined blood volume provision channel (see "capillary channel" in Figs. 1 and 5), fills with a fixed volume of blood enabled by the physical design of the test strip. A visual cue of blood filling the channel to know when a sufficient volume of blood has entered the test strip. For example, the user may rely on the visual cue of blood filling the channel to know when a sufficient volume of blood has entered the test strip. The volume of blood tested may be of high importance as the test strip is measuring a raw signal dependent on the total amount of blood where too much or too little blood would skew the results. However, as the fixed volume is limited by the channel and overall test strip design, overfilling the channel by user error is a reduced concern. When the channel is full of blood, the volume of blood within the channel remains fixed for a length of time that allows the test strip to be held to the blood drop, such as a user holding the test strip to their finger, for a variable length of time without changing the volume of blood.

A feature that was unexpectedly useful to the performance of the disclosed test strips is somewhat counterintuitive and thus highly unexpected, because the feature is a relative ease of collapsibility (e.g., a lack of mechanical strength) when compared to hard plastic or other physically more robust systems. This relative ease of collapsibility may be viewed as a detriment in certain circumstances, as physical strain might damage the test strip, but in this case without wishing to be bound by any particular theory, the relative ease of collapsibility may have significant unexpected advantages in facilitating the provision of a fixed volume of blood. In other systems having more structural rigidity, a user could impart a significant amount of force onto a finger prick site, such that forces responsible for the proper flow of blood are disturbed. With a very rigid structure, the user could apply a force that causes problems with blood flow. As disclosed herein, the test strips have a physical structure and are composed of materials (e.g., paper layers and thin polymeric layers) that provide collapsibility when the test strip experiences a lengthwise force (e.g., a force pushing from the distal end of the test strip onto the proximal end of the test strip) that exceeds a predetermined value. Existing systems that are made of harder plastic or other rigid materials include complex internal structures, such as double valves with dissolvable membranes or complex chamber structures with vents, to account for this possibility where too much blood may be introduced into a system by virtue of application of excessive force.

An expanded view of an embodiment of the test strip is shown in Fig. 1. The base layer, Component 1, has at least one surface in contact with a dye separation material, Component 6. The base layer may have adhesive on at least one side to facilitate adhesion during assembly. The sample pad, Component 2, has at least one surface in contact with the base layer, Component 1, the dye separation material, Component 6, and a second spacer, Component 4. The sample pad, Component 2 in Fig. 1, includes a red blood cell lysing agent in a dry form and a boronic acid dye in a dry form. The defined blood volume provision layer, Component 5, has at least one surface in contact with the dye separation material, Component 6, the second spacer, Component 4, the sample pad, Component 2, and the first spacer, Component 7. Component 7 may have adhesive on at least one side. The defined blood volume provision layer extends the entire length of the test strip while the defined blood volume provision channel (e.g., capillary channel) extends roughly half the length of the entire test strip. The top layer serves as a cover to the test strip and may include a visual identification portal to facilitate optical observation of the defined blood volume provision channel. Table 1 below describes each component in an embodiment in roughly top to bottom order as shown in Fig. 1, Fig. 2, and Figs. 3A and 3B. Placing Component 7 above Component 5 may provide an improved optical readout by ensuring Component 6 is in contact with the optical readout window. This embodiment may allow for improved flow through the test strip due to increased contact between Component 6 and Component 2.

**Table 1**

| **Component Number** | **Component Name** |
|---|---|
| 3 | Cover/Top layer |
| 7 | First spacer |
| 5 | Blood volume provision layer |
| 4 | Second spacer |
| 6 | Dye separation material |
| 2 | Sample pad |
| 1 | Base |

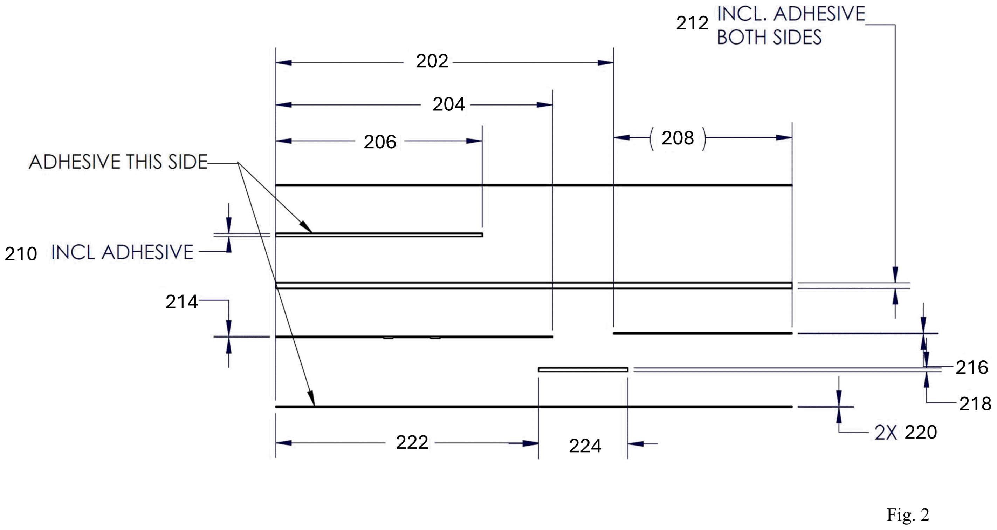

An expanded view of an embodiment of the test strip is shown in Fig. 2. Various relevant measurements are labeled as follows: distance from distal end of test strip to distal end of second spacer 202 (i.e., distance from distal end of test strip to the proximal end of the terminal opening - e.g., between 30 mm and 40 mm); dye separation material length 204 (e.g., between 25 mm and 35 mm); first spacer length 206 (e.g., between 10 mm and 30 mm); second spacer length 208 (e.g., between 15 mm and 40 mm); first spacer thickness 210 (e.g., between 0.05 mm and 0.30 mm); defined blood volume provision layer thickness 212 (e.g., between 0.20 mm and 1.00 mm); dye separation material thickness 214 (e.g., between 0.05 mm and 0.50 mm); first spacer thickness 216 (e.g., between 0.10 mm and 0.50 mm); sample pad thickness 218 (e.g., between 0.20 mm and 1.00 mm); top layer or bottom layer thickness 220 (e.g., between 0.05 mm and 0.20 mm); distance from distal end of test strip to distal end of sample pad 222 (e.g., between 20.0 mm and 40.0 mm); and sample pad length 224 (e.g., between 5.0 mm and 20.0 mm). Measurements provided herein should be taken to include typical manufacturing tolerances as would be appreciated by a person having ordinary skill in the art. Components 5 and 7 may have adhesive on at least one side.

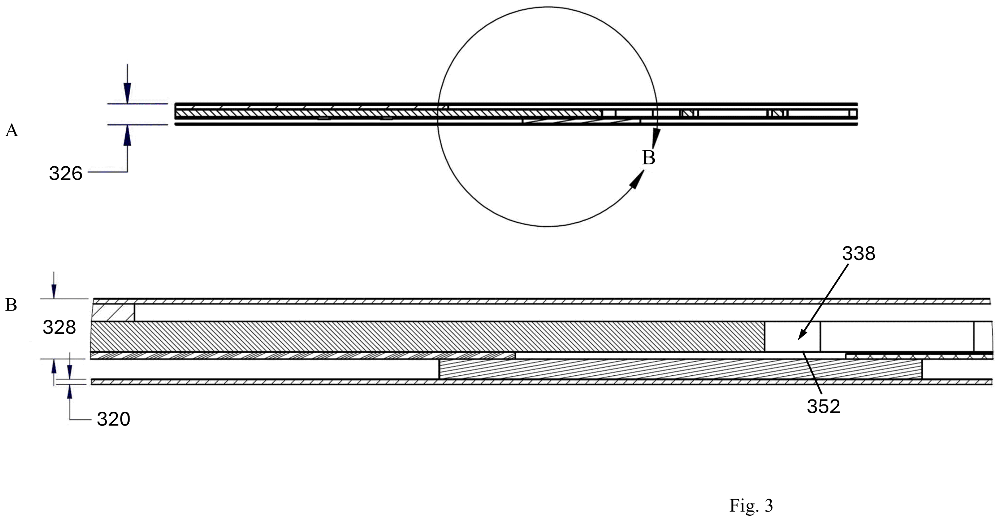

Fig. 3A shows a cross-section view of the test strip. Fig. 3B shows a zoomed in view of the circle indicated in Fig. 3A. Various relevant measurements are labeled as follows: test strip thickness 326 (e.g., between 1.5 mm and 2.5 mm); combined thickness of test strip minus sample pad and bottom layer 328 (e.g., between 1.0 mm and 2.25 mm); and bottom layer thickness 320. The test strip thickness 326 may be between 1.5 mm and 2.5 mm. The vertical gaps shown in Fig. 3B will be eliminated in the production process as would be appreciated by a person having ordinary skill in the art.

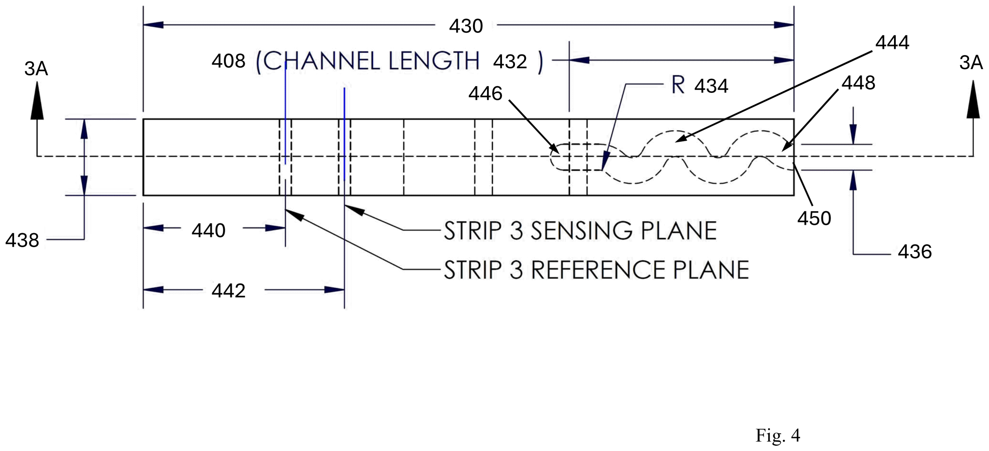

Fig. 4 shows a top down view of the test strip, showing internal structures including the defined blood volume provision layer in dotted lines. Various relevant measurements are labeled as follows: test strip length 430 (e.g., between 40 mm and 70 mm or between 50 mm and 60 mm); second spacer length 408; defined blood volume provision channel length 432 (i.e., length factoring curves in the channel - e.g., between 20 mm and 45 mm); channel radius of curvature 434 (e.g., between 0.3 mm and 1.5 mm); defined blood volume provision channel width 436 (e.g., between 1.00 mm and 5.00 mm); test strip width 438 (e.g., between 5.0 mm and 15.0 mm); a distance from the distal end of the test strip to a reference plane 440 (e.g., between 5 mm and 45 mm); a distance from the distal end of the test strip to a sensing plane 442 (e.g., between 5 mm and 45 mm). The defined blood volume provision layer has a defined blood volume provision channel which fills up with blood from the blood droplet engagement end on the right to a terminal end on the left. The blood droplet engagement end has a sealed engagement opening. The terminal end has a terminal opening at a bottom surface of the defined blood provision channel. The sample pad below the terminal opening is adapted to receive fluid from the terminal opening. The blood sample only migrates into the sample pad once the defined blood provision channel has the defined blood volume within. Fig. 3A is the cross-sectional view along the line labeled 3A in Fig. 4.

An expanded view of an additional embodiment of the test strip is shown in Fig. 5. The base, Component 1, has at least one surface in contact with a dye separation material, Component 6. The dye separation material is in contact with a first spacer, Component 7. The sample pad, Component 2 in Fig. 5, is in contact with a second spacer, Component 4, and a blood volume provision layer, Component 5. The defined blood volume provision layer extends the entire length of the test strip while the defined blood volume provision channel extends roughly half the length of the entire test strip. The top layer, Component 3, serves as a cover to the test strip and has a visual identification portal to facilitate optical observation of the defined blood volume provision channel. Table 1 above describes each component (though no longer in order from top to bottom) of the alternative embodiment as shown in Fig. 5, Fig. 6, and Figs. 7A and 7B.

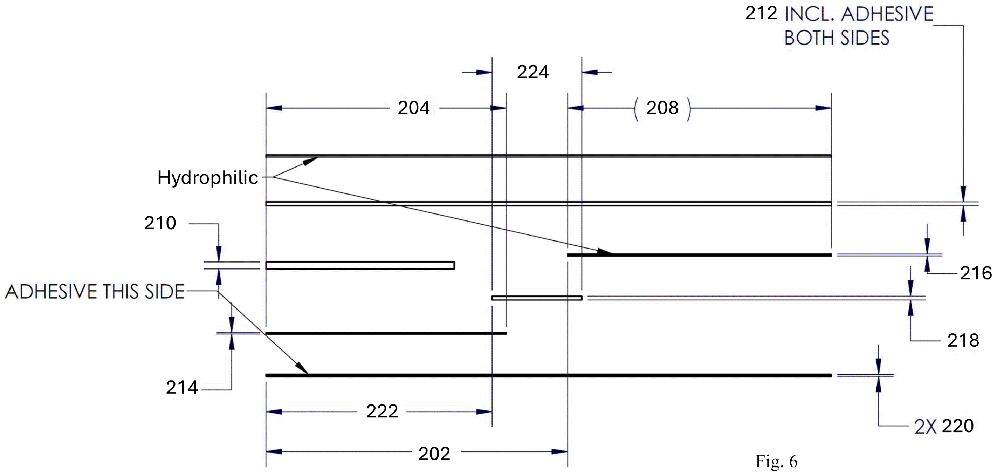

Fig. 6 shows a schematic of the additional embodiment of the HbA1c test strip. All measurements indicated are in mm. Relevant measurements are numbered with the same numbering system identified above with respect to Figs. 2-4. Components 5 and 7 may have adhesive on at least one side.

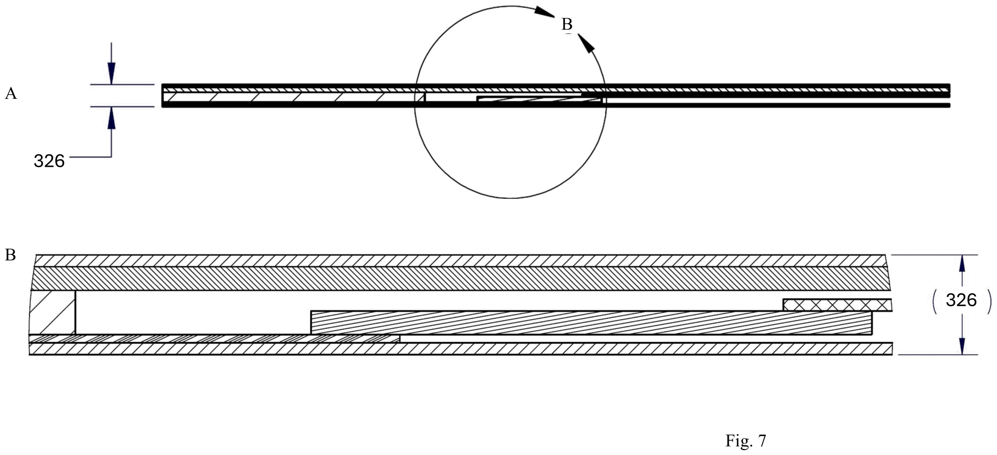

Fig. 7A shows a cross-section view of the additional embodiment of the test strip. Fig. 7B shows a zoomed in view of the circle indicated in Fig. 7A. The vertical gaps shown in Fig. 7B will be eliminated in the production process as would be appreciated by a person having ordinary skill in the art.

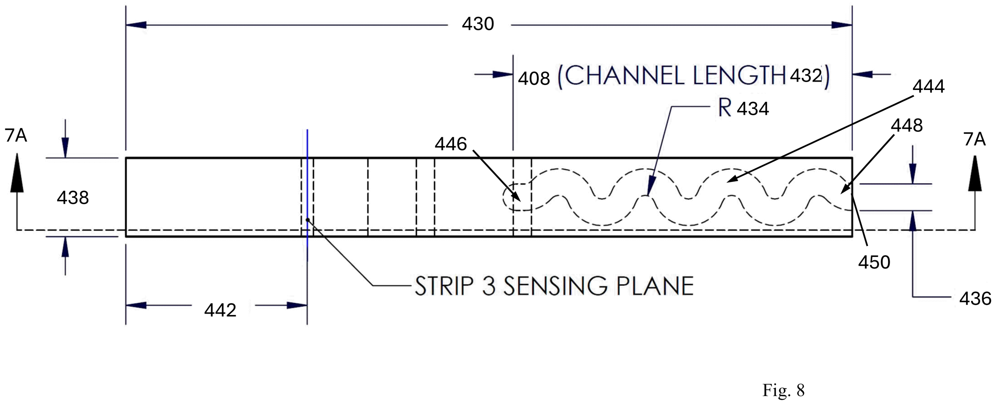

Fig. 8 shows a top down view of the additional embodiment of the test strip, showing internal structures including the defined blood volume provision layer in dotted lines. Fig. 7A is the cross-sectional view along the line labeled 7A in Fig. 8.

The test strip can have a test strip length 430 of between 30 mm and 150 mm. In some cases, the test strip length 430 can be at least 30 mm, at least 35 mm, at least 40 mm, at least 45 mm, at least 50 mm, at least 55 mm, or at least 75 mm. In some cases, the test strip length 430 can be at most 150 mm, at most 140 mm, at most 130 mm, at most 125 mm, at most 120 mm, at most 110 mm, at most 100 mm, at most 90 mm, at most 80 mm, at most 75 mm, at most 70 mm, at most 65 mm, at most 60 mm, at most 55 mm, or at most 50 mm.

The test strip can have a test strip width 438 of between 4.0 mm and 10.0 mm. In some cases, the test strip width 438 can be at least 4.0 mm, at least 5.0 mm, at least 6.0 mm, or at least 7.0 mm. In some cases, the test strip width 438 can be at most 10.0 mm, at most 9.0 mm, at most 8.0 mm, at most 7.0 mm, or at most 6.0 mm.

The test strip can have a test strip thickness 326 of between 1.5 mm and 2.5 mm. In some cases, the test strip thickness 326 can be at least 1.5 mm, at least 1.6 mm, at least 1.7 mm, at least 1.8 mm, at least 1.9 mm, or at least 2.0 mm. In some cases, the test strip thickness 326 can be at most 2.5 mm, at most 2.4 mm, at most 2.3 mm, at most 2.2 mm, at most 2.1 mm, or at most 2.0 mm.

The defined blood volume provision channel can be adapted to provide a precise volume of blood of between 5 µL and 100 µL or between 10 µL and 50 µL. In some cases, the precise volume of blood can be at least 5 µL, at least 10 µL, at least 15 µL, at least 20 µL, or at least 25 µL. In some cases, the precise volume of blood can be at most 50 µL, at most 45 µL, at most 40 µL, at most 35 µL, at most 30 µL, or at most 25 µL.

The defined blood volume provision layer can be made of a material that either hydrophilic or not hydrophilic and the defined blood volume provision channel may still properly function by virtue of having hydrophilic top and bottom surfaces. In some cases, the defined blood volume provision layer is made of polyethylene terephthalate (PET).

Referring to Figs. 4 and 8, the defined blood volume provision channel 444 has a blood droplet engagement end 446 and a terminal end 448. The blood droplet engagement end 446 has a sealed engagement opening 450 that is defined by the portions of defined blood volume provision layer and the layers atop and beneath the defined blood volume provision layer. Referring to Fig. 3B, the terminal end 338 includes a terminal opening 352 at a bottom surface of the defined blood volume provision channel.

A top and bottom surface of the defined blood volume provision channel 444 can be hydrophilic, while side surfaces of the defined blood volume provision channel 444 may be hydrophilic, non-hydrophilic, or hydrophobic. The hydrophilicity of the top and bottom surfaces can be introduced by way of a hydrophilic coating. Without wishing to be bound by any particular theory, it is believed that allowing the side walls of the defined blood volume provision channel 444 to be hydrophobic enhances the cost-effectiveness of the resulting test strip, because introducing hydrophilicity via the top and bottom surfaces can be achieved much more simply than the side surfaces, because the top and bottom surfaces can be introduced as long, flat strips of material.

The defined blood volume provision layer can have the test strip length 430 and the test strip width 438. A ratio of test strip length 430 to test strip width 438 may be between 5:1 and 15:1, including but not limited to between at least 5:1, at least 8:1, or at least 11:1, and at most 15:1, at most 12:1, at most 9:1, or at most 6:1. The defined blood volume provision channel may occupy between ⅓ and 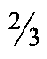 of the total length of the defined blood volume provision layer. The defined blood volume provision channel may have a length of the second spacer length 208, 408 when measured from end to end without considering curves in the tortuous pathway and the blood volume provision channel length 432 when in a straightened state. The second spacer length 208, 408 can be between 15 mm and 40 mm, including but not limited to, at least 15 mm, at least 20 mm, at least 25 mm, or at least 30 mm and at most 40 mm, at most 35 mm, at most 30 mm, at most 25 mm, or at most 20 mm. The defined blood volume provision channel has a height that is the blood volume provision layer thickness 212. The blood volume provision layer thickness 212 can be between 0.20 mm and 1.00 mm. In some cases, the blood volume provision layer thickness 212 can be at least 0.20 mm, at least 0.25 mm, at least 0.30 mm, at least 0.40 mm, or at least 0.50 mm. In some cases, the blood volume provision layer thickness 212 can be at most 1.00 mm, at most 0.95 mm, at most 0.90 mm, at most 0.80 mm, at most 0.75 mm, at most 0.70 mm, or at most 0.60 mm. The defined blood volume provision channel has a width that is the defined blood volume provision channel width 436. The defined blood volume provision channel width 436 can be between 1.00 mm and 5.00 mm, including but not limited to, at least 1.00 mm, at least 2.00 mm, or at least 3.00 mm and at most 5.00 mm, a most 4.00 mm, or at most 3.00 mm. The defined blood volume provision channel may have curves with a radius of between 0.3 mm and 1.5 mm, including but not limited to at least 0.3 mm, at least 0.5 mm, at least 0.8 mm, at least 1.1 mm, or at least 1.4 mm and at most 1.5 mm, at most 1.2 mm, at most 0.9 mm, or at most 0.6 mm. Surfaces of the defined blood volume provision channel may be hydrophilic, either intrinsically or by a coating.

The terminal opening 352 can have an area of between 0.5 mm² and 5.0 mm². In some cases, the terminal opening 352 can have an area of between 1.0 mm² and 3.0 mm². In some cases, the terminal opening 352 can have an area of between 1.5 mm² and 2.5 mm². In some cases, the terminal opening 352 can have an area of at least 0.5 mm², at least 1.0 mm², or at least 1.5 mm². In some cases, the terminal opening 352 can have an area of at most 5.0 mm², at most 4.0 mm², at most 3.0 mm², at most 2.5 mm², or at most 2.0 mm². The terminal opening 352 can have a height that is the blood volume provision layer thickness 212 and a width that is the blood volume provision channel width 436.

In some cases, the defined blood volume provision channel has a substantially consistent aspect ratio across its entire length. In certain aspects, the defined blood volume provision channel has an aspect ratio of width to height of between 1.5:1 and 10:1. In certain aspects, the defined blood volume provision channel has an aspect ratio of width to height of between 2:1 and 5:1. In certain aspects, the defined blood volume provision channel has an aspect ratio of width to height of between 2.5:1 and 4:1. In some cases, the defined blood volume provision channel has an aspect ratio of at least 1.5:1, at least 2:1, or at least 2.5:1. In some cases, the defined blood volume provision channel has an aspect ratio of at most 10:1, at most 8:1, at most 6:1, at most 5:1, or at most 4:1.

When a blood droplet contacts with the sealed engagement opening, such as when the user contacts a finger-prick blood droplet with the sealed engagement opening, the defined blood volume provision channel fills with a precise volume of the blood. The defined volume blood provision channel is adapted to accommodate a range of contact pressures, a range of contact angles, and a range of contact durations. The blood remains in the defined volume blood provision channel while contact between the finger-prick blood droplet and the sealed engagement opening is maintained.

Once the defined blood volume provision channel is full and the test strip is removed from the blood droplet, such as when the user removes the test strip from the blood droplet, fluid flows from the terminal opening onto the sample pad. The red blood cells within the blood sample are lysed on the sample pad to release the hemoglobin to be measured. The hemoglobin within the fluid interacts with the boronic acid dye to form dye-hemoglobin complexes and excess boronic acid dye. Fluid from the sample pad flows to the dye separation material. The dye separation material has different migration speeds for HbA1c bound to the boronic acid dye and the excess boronic acid dye, resulting in the dye separation material selectively binding to the excess boronic acid dye but allowing the dye-hemoglobin to migrate further down the dye separation material. Unexpectedly, the behavior resulting from the blood droplet being contacted with sealed engagement opening 450, such as the user contacting the finger-prick blood droplet with the sealed engagement opening 450, is independent of physical orientation of the test strip in space (i.e., proper function does not rely on a certain orientation relative to gravity).

The sample pad is beneath the terminal opening 352 and is adapted to receive fluid from the terminal opening 352. The sample pad and the terminal opening 352 are adapted to provide a wicking force to blood fully filling the defined blood volume provision channel, wherein blood fully filling the defined blood volume provision channel experiences a sealed capillary force when the sealed engagement opening is engaged with the blood droplet, wherein blood fully filling the defined blood volume provision channel experiences an unsealed capillary force when the sealed engagement opening is disengaged from the blood droplet. The wicking force, the sealed capillary force, and the unsealed capillary force are balanced to retain the blood filling the defined blood volume provision channel within the defined blood volume provision channel and to force flow of blood filling the defined blood volume provision channel into the sample pad upon disengagement. In certain aspects, between 10% and 20% of sample pad length overlaps with the terminal opening 352. The sample pad is an absorbent material where blood cells can enter into the material. Suitable materials include, but are not limited to, bound glass fiber or cellulose-based paper. One specific exemplary sample pad is Fusion 5 (available commercially from Cytiva, Marlborough, MA).

The red blood cell lysing agent in dry form can be any cell lysing agent that acts with sufficient speed so as to allow the red blood cell contents to interact with the boronic acid before entering the dye separation material. The lysing agent can be a surfactant, such as Triton X-100 or sodium dodecyl sulfate.

The boronic acid dye may be xylene cyanol 1,3-diamino-2-propanol (DAPOL) 4-carboxyphenylboronic acid (CPBA) or other boronic acid dyes with absorbance spectrum significantly different from hemoglobin (e.g., a blue dye). In one aspect, the boronic acid dye is made by conjugating a dye to 4-carboxyphenylboronicd acid. Examples of suitable boronic acid dyes are included in U.S. Patent No. 5,631,364, which is incorporated herein in its entirety by reference for all purposes. In one aspect, the boronic acid dye may be xylene cyanol 1,3-diamino-2-propanol (DAPOL) 4-carboxyphenylboronic acid (CPBA), which is a blue color. After the fluid has migrated down the dye separation material, the color of the dye separation material as a function of hemoglobin and dye-hemoglobin is optically interrogable. The red:blue color ratio, corresponding to hemoglobin:dye-hemoglobin, is correlated to the total hemoglobin:glycated hemoglobin ratio present in the blood sample.

The dye separation material is one exemplary downstream component for an HbA1c test strip in accordance with aspects of the present disclosure. Other tests besides the HbA1c test may utilize the dye separation material or a material having similar function. The dye separation material is capable of operating under principles of selective interaction, under principles of size exclusion, or a combination thereof. In some cases, the dye separation material provides at least 2 mm, at least 3 mm, or at least 5 mm of separation between the HbA1c having the boronic acid dye bound thereto and unbound boronic acid dye. In some cases, the dye separation material has a slower migration speed for the unbound boronic acid dye than for the HbA1c having the boronic acid dye bound thereto.

Physical orientation of the dye separation material relative to other components may be important to function of the test strip. For example, physically orienting the dye separation material to overlap with the sample pad may be beneficial to function of the test strip by facilitating blood flow. In some cases, the dye separation material overlaps atop the sample pad (Figs. 1-4). In some cases, the dye separation material overlaps beneath the sample pad (Figs. 5-8). As another example, the amount of overlap between the dye separation material and the sample pad may be beneficial to function of the test strip by facilitating blood flow. In some cases, the overlap between the dye separation material and the sample pad can result in between 10% and 20% of sample pad length (or sample pad area/volume) overlapping with the dye separation material. The dye separation material and sample pad may be in fluid contact by way of an area of vertical overlap between the dye separation material and the sample pad. The area of vertical overlap between the dye separation material and the sample pad can be between 5.0 mm² and 20.0 mm². In some cases, the area of vertical overlap between the dye separation material and the sample pad can be at least 5.0 mm², at least 6.0 mm², or at least 7.5 mm². In some cases, the area of vertical overlap between the dye separation material and the sample pad can be at most 20.0 mm², at most 15.0 mm² or at most 12.5 mm².

In the case of selective interaction, chemical and/or charge-based interactions can cause the bound and unbound dye to have different migration speeds through the dye separation material. In some cases, the dye separation material can be a material containing vicinal diols (e.g., 1,2-diol groups). These vicinal diols could be native to the material (e.g., cellulose with a low degree of polymerization) or introduced to a material via some form of treatment (e.g., treatment with an alginic acid salt such as sodium alginate). In some cases, the material can be negatively charged. The dye separation material may be nitrocellulose, porous papers (e.g., cotton linter paper), and the like.

In the case of size-based dye separation material, the unbound dye will migrate further than bound dye because it is physically a much smaller entity. A skilled artisan will recognize suitable size-based dye separation materials in the art. Examples of suitable size-based separation materials include, but are not limited to, hydrophilic polyethersulfone membranes with ~5 µm pore size or the like. Suitable size-based separation materials are described in U.S. Patent Application Pub. No. 2019/0232287, which is incorporated herein in its entirety by reference for all purposes.

The base layer can be composed of a variety of materials that can suitably perform the necessary functions of the base layer. The base layer serves to provide some degree of structural stability and to serve as a barrier to prevent contamination of inner layers. In some aspects, the base layer is transparent at least at a location where measurements are taken in one or more downstream components (e.g., dye separation material). In some cases, the base layer is composed of a polymeric material. In some cases, the base layer is composed of polyester. In some cases, the base layer can be composed of polyvinyl chloride. The base layer may be a clear polyester film.

The test strip can include a sensing plane. The test strip can be substantially transparent from at least one direction to the dye separation material at the sensing plane. This allows optical interrogation of the dye separation material at the sensing plane. The sensing plane can be located at the distance at which the bound dye propagates.

While portability and usability of these test strips are helped by their compact design and slim profile, the features described herein may be applicable and may still properly function if the device is scaled up or down in size dimensions while retaining the relative dimensions disclosed herein. As such, the present disclosure expressly contemplates embodiments of a test strip having physical dimensions that are 0.5x, 0.6x, 0.7x, 0.75x, 0.8x, 0.9x, 1.1x, 1.2x, 1.3x, 1.5x, 1.75x, or 2.0x the specific physical dimensions disclosed herein. In some cases, the test strip can have physical dimensions that are at least 0.5x, at least 0.6x, at least 0.7x, at least 0.75 x, at least 0.8x, or at least 0.9x and at most 1.0x, at most 0.95x, at most 0.9x, at most 0.8x, at most 0.75x, at most 0.7x, or at most 0.6x the specific physical dimensions disclosed herein. In some cases, the test strip can have physical dimensions that are at least 1.0x, at least 1.1x, at least 1.2x, at least 1.3x, at least 1.4x, at least 1.5x, or at least 1.75x and at most 1.9x, at most 1.8x, at most 1.7x, at most 1.6x, at most 1.5, or at most 1.25x the specific physical dimensions disclosed herein. Without wishing to be bound by any particular theory, it is believed that the test strip could properly function so long as the size dimensions of the test strip remain within a window where capillary forces remain principally responsible for movement of blood through the defined blood volume provision channel.

In another aspect, an HbA1c test kit for at-home use includes the HbA1c test strip described herein with instructions for at-home use. The HbA1c test kit may include an optical analysis device which can sense signal from HbA1c having the boronic acid dye bound thereto signal location.

In one aspect, a method of at-home HbA1c testing includes steps a) to d). Step a), if present, includes pricking a finger of a subject to produce a finger-prick blood droplet. Step a) is optional. Step a) may comprise providing a blood droplet, such as a finger-prick blood droplet. Step b) includes contacting the finger-prick drop of blood to the blood droplet engagement end of the HbA1c test strip described herein. Step c) includes measuring a signal from a location having the HbA1c having the boronic acid dye bound thereto. Step d) includes generating a report including HbA1c concentration from the blood based on the signal generated in step c).

With respect to the specific spectroscopic methods of measuring HbA1c concentration within the blood, a skilled artisan recognizes a variety of methods for acquiring and processing data to determine HbA1c concentration. One exemplary system and method for acquiring and processing data to determine HbA1c concentration is International Patent Application Pub. No. WO 2019/152508 A1, which is incorporated herein in its entirety by reference for all purposes.

In another aspect, a method of making an HbA1c test strip includes steps a) and b). Step a) includes assembling a layered structure including: a base layer, a sample pad and optional dye separation layer positioned atop the base layer, the sample pad and optional dye separation layer comprising a sample pad and optionally a dye separation material; optional spacer layers and optional spacers within the other layers; a defined blood volume provision layer positioned atop the sample pad and dye separation layer, the defined blood volume provision layer comprising a plurality of defined blood volume provision channels each having a blood droplet engagement end and a terminal end, wherein the blood droplet engagement end has a sealed engagement opening, wherein the terminal end has a terminal opening at a bottom surface of the defined blood volume provision channel; and a top layer positioned atop the defined blood volume provision layer. Step b) includes cutting the layered structure into individual test strips ensuring each individual test strip has at least one defined blood volume provision channel. The method of making test strips may include making sheets of test strips with a plurality of test strips repeating in both length and width directions. The sheets of test strips may be rolled up due to flexible backing and/or transported as sheets to be cut by a distributor, doctors office, hospital, end user, or the like. Sheets may be produced using roll to roll production, laminating, sheet-to-sheet, sheets-on-shuttle, and similar industrial processes.

The test strip contains evidence of this method of manufacturing within its physical structure and a skilled artisan will recognize that the test strips described herein can be cut into individual test strips from a series of laterally-extending repeat units. The uncut sheets of test strips can include various registration features to ensure that the test strips are properly cut into individual test strips with a complete defined blood volume provision channel.

A skilled artisan will recognize that the methods and test strips disclosed herein possess significant improvements relative to the state of the art, particularly when it comes to cost and ease of manufacturing. While previous efforts at providing a defined blood volume have included more expensive production methods, such as injection molding, the disclosed methods utilize methods of assembling layers of materials that are generally rectangular in shape and which are assembled with relative ease. In most cases, the lone physical element that is not strictly rectangular in shape and uniform in material composition and thickness is the defined blood volume provision layer, which includes the defined blood volume provision channel. A plurality of defined blood volume provision channels that are properly spaced can be introduced into a material that is generally rectangular in shape, and the entirety of the internal structural complexity of the test strip can be accounted for by this channel and the mechanical assembly of the other substantially rectangular-shaped layers.

All references, including publications, patent applications, and patents, cited herein are hereby incorporated by reference to the same extent as if each reference were individually and specifically indicated to be incorporated by reference and were set forth in its entirety herein.

Preferred aspects of this invention are described herein, including the best mode known to the inventors for carrying out the invention. Variations of those preferred aspects may become apparent to those of ordinary skill in the art upon reading the foregoing description. The inventors expect a person having ordinary skill in the art to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

While the invention has been illustrated and described in detail in the foregoing drawings and description, the same is to be considered as illustrative and not restrictive in character, it being understood that only illustrative embodiments thereof have been shown and described and that all changes and modifications that come within the spirit of the invention are desired to be protected. For example, any of the features or functions of any of the embodiments disclosed herein may be incorporated into any of the other embodiments disclosed herein.

The following examples illustrate some embodiments and aspects of the invention. It will be apparent to those skilled in the relevant art that various modifications, additions, substitutions, and the like can be performed without altering the spirit or scope of the invention, and such modifications and variations are encompassed within the scope of the invention as defined in the claims which follow. The following examples do not in any way limit the invention.

### EXAMPLES

### Example 1:

Test strips according to the designs described above were built using a 50 x 6.5 x 0.5mm (LxWxH) PET backing material with adhesive on the bottom side. A small rectangular shaped gap was cut into the material 21 mm long and 1.5 mm wide leaving 2.5mm of PET remaining on each side. An adhesive layer was added to the top side of the PET layer and a clear, hydrophilic layer was attached creating the top of the capillary channel. On the bottom side, nitrocellulose was laid down, followed by the sample pad (Cytiva Fusion 5) overlapping the nitrocellulose by 2mm. The blood separator overlaps into the channel by 2mm. A clear hydrophilic layer (3M 9984) was attached to the bottom adhesive of the PET backing and overlaps the fusion 5 by 6mm. A clear film with adhesive was used to cover the length of the strip. 20-25uL of blood, formed in a drop, was applied to the open port in the capillary channel. The blood traveled through the channel via capillary force and is delivered to the sample pad. Blood layer absorbs the blood and continues to pull the rest of the blood into the pad. The sample pad filters out the red blood cells and plasma flows down the nitrocellulose, leaving the red blood cells in the pad, thereby confirming visually that spectroscopic interrogation was accessible. If less than 20 uL of blood was applied to the capillary channel, the sample pad did not wet.

### EQUIVALENTS AND SCOPE

The recitation of a listing of elements in any definition of a variable herein includes definitions of that variable as any single element or combinations (or subcombinations) of listed elements. The recitation of an embodiment herein includes that embodiment as any single embodiment or in combination with any other embodiments or portions thereof. Those skilled in the art will recognize or be able to ascertain using no more than routine experimentation, many equivalents to the specific embodiments of the invention described herein.

In addition to the features described above and elsewhere herein, the present disclosure also includes the following clauses:
Clause 1. A test strip for at-home use comprising: a top layer; a defined blood volume provision layer beneath the top layer, the defined blood volume provision layer comprising a defined blood volume provision channel having a blood droplet engagement end and a terminal end, wherein the blood droplet engagement end has a sealed engagement opening, wherein the defined blood volume provision channel has at least one hydrophilic inner surface, wherein the terminal end has a terminal opening at a bottom surface of the defined blood volume provision channel; and a sample pad beneath the terminal opening and adapted to receive fluid from the terminal opening, wherein the sample pad optionally comprises chemical agents in dry form, wherein contacting a finger-prick blood droplet with the sealed engagement opening, such as a user contacting a finger-prick blood droplet with the sealed engagement opening, causes the defined blood volume provision channel to fill with a precise volume of the blood, wherein the blood remains in the defined blood volume provision channel while contact between the finger-prick blood droplet and the sealed engagement opening is maintained, wherein the blood flows into the sample pad following separation between the finger-prick blood droplet and the sealed engagement opening, wherein the defined blood volume provision channel is adapted to accommodate a range of contact pressures and a range of contact durations.
Clause 2. A hemoglobin A1c (HbA1c) test strip for at-home use comprising: a top layer; a defined blood volume provision layer beneath the top layer, the defined blood volume provision layer comprising a defined blood volume provision channel having a blood droplet engagement end and a terminal end, wherein the blood droplet engagement end has a sealed engagement opening, wherein the defined blood volume provision channel has at least one hydrophilic inner surface, wherein the terminal end has a terminal opening at a bottom surface of the defined blood volume provision channel; a sample pad beneath the terminal opening and adapted to receive fluid from the terminal opening, wherein the sample pad comprises a red blood cell lysing agent in dry form and a boronic acid dye in dry form; and a dye separation material in fluid contact with the sample pad, such that fluid from the sample pad flows into the dye separation material, wherein the dye separation material has different migration speeds for HbA1c having the boronic acid dye bound thereto and unbound boronic acid dye, wherein contacting a finger-prick blood droplet with the sealed engagement opening, such as a user contacting a finger-prick blood droplet with the sealed engagement opening, causes the defined blood volume provision channel to fill with a precise volume of the blood, wherein the blood remains in the defined blood volume provision channel while contact between the finger-prick blood droplet and the sealed engagement opening is maintained, wherein the blood flows into the sample pad following separation between the finger-prick blood droplet and the sealed engagement opening, wherein the defined blood volume provision channel is adapted to accommodate a range of contact pressures and a range of contact durations.
Clause 3. The test strip of any one of the preceding clauses, wherein the precise volume of blood is between 5 µL and 100 µL or between 10 µL and 50 µL.
Clause 4. The test strip of any one of the preceding clauses, wherein the test strip has a test strip length of between 30 and 150 mm.
Clause 5. The test strip of any one of the preceding clauses, wherein the test strip has a test strip width of between 4.0 mm and 10.0 mm.
Clause 6. The test strip of any one of the preceding clauses, wherein the test strip has a test strip thickness of between 1.5 mm and 2.5 mm.
Clause 7. The test strip of any one of the preceding clauses, wherein the defined blood volume provision layer is made of polyethylene terephthalate (PET).
Clause 8. The test strip of any one of the preceding clauses, wherein the defined blood volume provision channel has a tortuous pathway, thereby enhancing volume of the channel relative to a comparison linear pathway having otherwise identical dimensions.
Clause 9. The test strip of any one of the preceding clauses, wherein the behavior resulting from contacting the finger-prick blood droplet with the sealed engagement opening, such as the user contacting the finger-prick blood droplet with the sealed engagement opening, is independent of physical orientation of the test strip in space.
Clause 10. The test strip of any one of the preceding clauses, wherein the sample pad and the terminal opening are adapted to provide a wicking force to blood fully filling the defined blood volume provision channel, wherein blood fully filling the defined blood volume provision channel experiences a sealed capillary force when the sealed engagement opening is engaged with the blood droplet, wherein blood fully filling the defined blood volume provision channel experiences an unsealed capillary force when the sealed engagement opening is disengaged from the blood droplet, wherein the wicking force, the sealed capillary force, and the unsealed capillary force are balanced to retain the blood filling the defined blood volume provision channel within the defined blood volume provision channel and to force flow of blood filling the defined blood volume provision channel into the sample pad upon disengagement.
Clause 11. The test strip of any one of the preceding clauses, wherein the terminal opening has an area of between 0.5 mm2 and 5.0 mm2, including but not limited to, between 1.0 mm2 and 3.0 mm2 or between 1.5 mm2 and 2.5 mm2.
Clause 12. The test strip of any one of the preceding clauses, wherein the defined blood volume provision channel has a substantially consistent aspect ratio across its entire length.
Clause 13. The test strip of any one of the preceding clauses, wherein the defined blood volume provision channel has an aspect ratio of width to height of between 1.5:1 and 10:1, including but not limited to, between 2:1 and 5:1 or between 2.5:1 and 4:1.
Clause 14. The test strip of any one of the preceding clauses, wherein the bottom surface of the defined blood volume provision channel is defined by a spacer layer beneath the defined blood volume provision layer.
Clause 15. The test strip of any one of the preceding clauses, wherein the top layer comprises a visual identification portal positioned above the terminal opening, which allows visual identification when the defined blood volume provision channel has been filled with the precise volume of the blood, such as allowing the user to visually identify when the defined blood volume provision channel has been filled with the precise volume of the blood.
Clause 16. The test strip of any one of the preceding clauses, wherein the defined blood volume provision channel has a channel height of between 0.20 mm and 1.00 mm.
Clause 17. The test strip of any one of the preceding clauses, wherein the defined blood volume provision channel has a channel width of between 1.00 mm and 5.00 mm.
Clause 18. The test strip of any one of the preceding clauses, wherein the sample pad has a thickness of between 0.20 mm and 1.00 mm.
Clause 19. The test strip of any one of the preceding clauses, wherein between 10% and 20% of sample pad length overlaps with the terminal opening.
Clause 20. The test strip of any one of clauses 2 to the immediately preceding clause, wherein the dye separation material is in fluid contact with the sample pad by way of an area of vertical overlap with the sample pad.
Clause 21. The test strip of the immediately preceding clause, wherein between 10% and 20% of sample pad length overlaps with the dye separation material.
Clause 22. The test strip of either two of the immediately preceding clauses, wherein the area of vertical overlap is between 5.0 mm2 and 20.0 mm2, including but not limited to, between 6.0 mm2 and 15.0 mm2 or between 7.5 mm2 and 12.5 mm2.
Clause 23. The test strip of any one of the three immediately preceding clauses, wherein the dye separation material overlaps atop the sample pad.
Clause 24. The test strip of clause 20 or 22, wherein the dye separation material overlaps beneath the sample pad.
Clause 25. The test strip of any one of clauses 2 to the immediately preceding clause, wherein the boronic acid dye is xylene cyanol 1,3-diamino-2-propanol (DAPOL) 4-carboxyphenylboronic acid (CPBA).
Clause 26. The test strip of any one of clauses 2 to the immediately preceding clause, wherein the dye separation material provides at least 2 mm, at least 3 mm, or at least 5 mm of separation between the HbA1c having the boronic acid dye bound thereto and the unbound boronic acid dye.
Clause 27. The test strip of any one of clauses 2 to the immediately preceding clause, wherein the dye separation material has a slower migration speed for the unbound boronic acid dye than for the HbA1c having the boronic acid dye bound thereto.
Clause 28. The test strip of any one of clauses 2 to the immediately preceding clause, wherein the dye separation material is negatively charged.
Clause 29. The test strip of any one of clauses 2 to the immediately preceding clause, wherein the dye separation material is nitrocellulose.
Clause 30. The test strip of any one of clauses 2 to the immediately preceding clause, wherein the test strip includes a sensing plane, wherein the test strip is substantially transparent from at least one direction to the dye separation material at the sensing plane, thereby allowing optical interrogation of the dye separation material at the sensing plane.
Clause 31. The test strip of any one of the preceding clauses, wherein the test strip is cut into an individual test strip from a series of laterally-extending repeat units.
Clause 32. An HbA1c test kit for at-home use comprising the HbA1c test strip of any one of the preceding clauses and instructions for at-home use.
Clause 33. The HbA1c test kit of the immediately preceding clause, further comprising an optical analysis device adapted to sense signal from an HbA1c having the boronic acid dye bound thereto at the signal location.
Clause 34. A method of at-home hemoglobin A1c (HbA1c) testing, the method comprising the following steps: a) providing a finger-prick blood droplet, such as pricking a finger of a subject to produce a finger-prick blood droplet; b) contacting the finger-prick blood droplet to the blood droplet engagement end of the HbA1c test strip or test kit of any one of the preceding clauses; c) measuring a signal from a location having the HbA1c having the boronic acid dye bound thereto; d) generating a report including a HbA1c concentration from the blood based on the signal measured in step c).
Clause 35. A method of making a test strip, the method comprising: a) assembling a layered structure including: a base layer; a sample pad and optional dye separation layer positioned atop the base layer, the sample pad and optional dye separation layer comprising a sample pad and optionally a dye separation material; optional spacer layers and optional spacers within the other layers; a defined blood volume provision layer positioned atop the sample pad and dye separation layer, the defined blood volume provision layer comprising a plurality of defined blood volume provision channels each having a blood droplet engagement end and a terminal end, wherein the blood droplet engagement end has a sealed engagement opening, wherein the terminal end has a terminal opening at a bottom surface of the defined blood volume provision channel; and a top layer positioned atop the defined blood volume provision layer; and b) cutting the layered structure into individual test strips, wherein each individual test strip includes at least one defined blood volume provision channel.

The scope of the present invention is not intended to be limited to the above Description, but rather is as set forth in the following claims:

## Claims

1. A test strip for at-home use comprising:
a top layer;
a defined blood volume provision layer beneath the top layer, the defined blood volume provision layer comprising a defined blood volume provision channel having a blood droplet engagement end and a terminal end, wherein the blood droplet engagement end has a sealed engagement opening, wherein the defined blood volume provision channel has at least one hydrophilic inner surface, wherein the terminal end has a terminal opening at a bottom surface of the defined blood volume provision channel; and
a sample pad beneath the terminal opening and adapted to receive fluid from the terminal opening, wherein the sample pad optionally comprises chemical agents in dry form,
wherein contacting a finger-prick blood droplet with the sealed engagement opening causes the defined blood volume provision channel to fill with a precise volume of the blood, wherein the blood remains in the defined blood volume provision channel while contact between the finger-prick blood droplet and the sealed engagement opening is maintained, wherein the blood flows into the sample pad following separation between the finger-prick blood droplet and the sealed engagement opening, wherein the defined blood volume provision channel is adapted to accommodate a range of contact pressures and a range of contact durations.

2. A hemoglobin A1c (HbA1c) test strip for at-home use comprising:
a top layer;
a defined blood volume provision layer beneath the top layer, the defined blood volume provision layer comprising a defined blood volume provision channel having a blood droplet engagement end and a terminal end, wherein the blood droplet engagement end has a sealed engagement opening, wherein the defined blood volume provision channel has a at least one hydrophilic inner surface, wherein the terminal end has a terminal opening at a bottom surface of the defined blood volume provision channel;
a sample pad beneath the terminal opening and adapted to receive fluid from the terminal opening, wherein the sample pad comprises a red blood cell lysing agent in dry form and a boronic acid dye in dry form; and
a dye separation material in fluid contact with the sample pad, such that fluid from the sample pad flows into the dye separation material, wherein the dye separation material has different migration speeds for HbA1c having the boronic acid dye bound thereto and unbound boronic acid dye,
wherein contacting a finger-prick blood droplet with the sealed engagement opening causes the defined blood volume provision channel to fill with a precise volume of the blood, wherein the blood remains in the defined blood volume provision channel while contact between the finger-prick blood droplet and the sealed engagement opening is maintained, wherein the blood flows into the sample pad following separation between the finger-prick blood droplet and the sealed engagement opening, wherein the defined blood volume provision channel is adapted to accommodate a range of contact pressures and a range of contact durations,
optionally wherein the boronic acid dye is xylene cyanol 1,3-diamino-2-propanol (DAPOL) 4-carboxyphenylboronic acid (CPBA).

3. The test strip of any one of the preceding claims, wherein the precise volume of blood is between 5 µL and 100 µL or between 10 µL and 50 µL.

4. The test strip of any one of the preceding claims, wherein the test strip has at least one of a test strip length of between 30 and 150 mm, a test strip width of between 4.0 mm and 10.0 mm, and/or a test strip thickness of between 1.5 mm and 2.5 mm.

5. The test strip of any one of the preceding claims, wherein the defined blood volume provision layer is made of polyethylene terephthalate (PET).

6. The test strip of any one of the preceding claims, wherein the defined blood volume provision channel has a tortuous pathway, thereby enhancing volume of the channel relative to a comparison linear pathway having otherwise identical dimensions.

7. The test strip of any one of the preceding claims, wherein the behavior resulting from contacting the finger-prick blood droplet with the sealed engagement opening, such as the user contacting the finger-prick blood droplet with the sealed engagement opening, is independent of physical orientation of the test strip in space.

8. The test strip of any one of the preceding claims, wherein the sample pad and the terminal opening are adapted to provide a wicking force to blood fully filling the defined blood volume provision channel, wherein blood fully filling the defined blood volume provision channel experiences a sealed capillary force when the sealed engagement opening is engaged with the blood droplet, wherein blood fully filling the defined blood volume provision channel experiences an unsealed capillary force when the sealed engagement opening is disengaged from the blood droplet, wherein the wicking force, the sealed capillary force, and the unsealed capillary force are balanced to retain the blood filling the defined blood volume provision channel within the defined blood volume provision channel and to force flow of blood filling the defined blood volume provision channel into the sample pad upon disengagement.

9. The test strip of any one of the preceding claims, wherein the terminal opening has an area of between 0.5 mm² and 5.0 mm², including but not limited to, between 1.0 mm² and 3.0 mm² or between 1.5 mm² and 2.5 mm².

10. The test strip of any one of the preceding claims, wherein the defined blood volume provision channel has a substantially consistent aspect ratio across its entire length and/or an aspect ratio of width to height of between 1.5:1 and 10:1, including but not limited to, between 2:1 and 5:1 or between 2.5:1 and 4:1.

11. The test strip of any one of the preceding claims, wherein the bottom surface of the defined blood volume provision channel is defined by a spacer layer beneath the defined blood volume provision layer.

12. The test strip of any one of the preceding claims, wherein the top layer comprises a visual identification portal positioned above the terminal opening, which allows visual identification of when the defined blood volume provision channel has been filled with the precise volume of the blood, such as allowing the user to visually identify when the defined blood volume provision channel has been filled with the precise volume of the blood.

13. The test strip of any one of the preceding claims, wherein the defined blood volume provision channel has a channel height of between 0.20 mm and 1.00 mm or between 1.00 mm and 5.00 mm.

14. The test strip of any one of the preceding claims, wherein the sample pad has a thickness of between 0.20 mm and 1.00 mm and/or wherein between 10% and 20% of sample pad length overlaps with the terminal opening.

15. The test strip of any one of claims 2 to the immediately preceding claim, wherein the dye separation material is in fluid contact with the sample pad by way of an area of vertical overlap with the sample pad, optionally wherein between 10% and 20% of sample pad length overlaps with the dye separation material, optionally wherein the area of vertical overlap is between 5.0 mm² and 20.0 mm², including but not limited to, between 6.0 mm² and 15.0 mm² or between 7.5 mm² and 12.5 mm², optionally wherein the dye separation material overlaps atop the sample pad, optionally wherein the dye separation material overlaps beneath the sample pad,
optionally wherein the dye separation material provides at least 2 mm, at least 3 mm, or at least 5 mm of separation between the HbA1c having the boronic acid dye bound thereto and the unbound boronic acid dye, wherein the dye separation material has a slower migration speed for the unbound boronic acid dye than for the HbA1c having the boronic acid dye bound thereto, wherein the dye separation material is negatively charged, and/or wherein the dye separation material is nitrocellulose, optionally wherein the test strip includes a sensing plane, wherein the test strip is substantially transparent from at least one direction to the dye separation material at the sensing plane, thereby allowing optical interrogation of the dye separation material at the sensing plane.
